# EUROPEAN PATENT SPECIFICATION

(11) **EP 4 637 556 B1**
(45) Date of publication and mention of the grant of the patent: **18.11.2026**
(21) Application number: 23833776.0
(22) Date of filing: 18.12.2023
(51) Int. Cl.: A61B 5/1455, A61B 5/1495, A61B 5/00, A61B 90/00, G06T 7/00, G06T 7/33, G06T 7/30, G06T 7/80

(54) **ALIGNING SIMULTANEOUSLY ACQUIRED IMAGES**
AUSRICHTUNG VON GLEICHZEITIG AUFGENOMMENEN BILDERN
ALIGNEMENT D'IMAGES ACQUISES SIMULTANÉMENT

(30) Priority: 22.12.2022 EP 22216214
(43) Date of publication of application: 29.10.2025
(73) Proprietor: Neko Health AB, 182 33 Danderyd (SE)
(72) Inventor: WINDÅ, Mattias, 185 39 Vaxholm (SE); ARVIDSSON, Magnus, 192 70 Sollentuna (SE); RÖÖS, Magnus, 753 33 Uppsala (SE); TINGSTAM, Olle, 183 56 Täby (SE); JOHANSSON, Henrik, 115 45 Stockholm (SE)
(74) Representative: Kransell & Wennborg KB
(86) International application number: PCT/EP2023/086447
(87) International publication number: WO 2024/133135

(56) References cited:
- WO-A1-2022/015597
- US-A1- 2006 029 291
- US-A1- 2007 038 122
- US-A1- 2022 086 416
- LO PRESTI LILIANA ET AL: "Multi-modal Medical Image Registration by Local Affine Transformations :", PROCEEDINGS OF THE 7TH INTERNATIONAL CONFERENCE ON PATTERN RECOGNITION APPLICATIONS AND METHODS, 1 January 2018 (2018-01-01), pages 534 - 540, XP093134386, ISBN: 978-989-7582-76-9, Retrieved from the Internet <URL:https://pdfs.semanticscholar.org/7b2a/b9264ca31939be3a5f5f2edea710dfb5b7c0.pdf> [retrieved on 20240223], DOI: 10.5220/0006656405340540
- EDOARDO ARDIZZONE ET AL: "Multi-modal non-rigid registration of medical images based on mutual information maximization", 14TH INTERNATIONAL CONFERENCE ON IMAGE ANALYSIS AND PROCESSING, 2007. ICIAP 2007, 10-13 SEPT. 2007 - MODENA, ITALY, IEEE, PISCATAWAY, NJ, USA, 1 September 2007 (2007-09-01), pages 743 - 750, XP031152428, ISBN: 978-0-7695-2877-9

## Description

### TECHNICAL FIELD

The present disclosure relates to a method of aligning images of a sample.

### BACKGROUND

EP 2 501 288 discloses an apparatus for determining subsurface optical properties of a sample of turbid media over an area of the sample. The apparatus comprises a source configured to expose the area of the sample to a plurality of structured illuminations, a detection system configured to collect optical signals emitted from the surface of the illuminated area, and a signal processor operably coupled to the detection system and configured to reconstruct optical data from the collected optical signals corresponding to the plurality of structured illuminations, wherein the collected optical signals from which optical data is reconstructed includes only one optical signal per each of the plurality of structured illuminations.

US 2022/086416 discloses an optical imaging system for capturing both 3D topography images and fluorescence images of a target object. Two separate cameras may be used which both detect the same frequency to avoid obstruction of view.

However, to determine the subsurface optical properties, optical signals at different wavelength ranges need to be collected, by using optical filters in front of a CCD camera, e.g. on a filter wheel.

### SUMMARY

It has now been realized that to acquire sequential images of a sample at different wavelengths takes long time, increasing the risk of the sample moving between images during the acquiring, especially if the sample is live tissue. It is herein proposed to instead acquire images at different respective wavelengths simultaneously by a plurality of cameras, e.g. arranged side by side in a detector. Then, however, each camera has a slightly different view of the sample, resulting in a problem of aligning the acquired images. Since each sample may have a distinct three-dimensional shape, this aligning may need to be specific for each sample.

According to an aspect of the present invention, there is provided a method of aligning simultaneously acquired images of a sample irradiated with a predetermined pattern. The method comprises, with said predetermined pattern, irradiating an area of the sample with electromagnetic radiation, typically including wavelengths absorbable by molecules present within the sample, below a surface of the sample. The method also comprises, by each of a plurality of cameras simultaneously, during the irradiating of the sample with the predetermined pattern, acquiring an image of the sample area in a wavelength range specific to that camera. Preferably, the wavelength range specific to that camera is different or distinct from the respective wavelength ranges of the other cameras, implying that each of the cameras is arranged to detect electromagnetic radiation in a wavelength range which is specific for that camera and which is different from, and in some different embodiments overlapping or not overlapping with, the respective wavelength ranges of the other cameras of the plurality of cameras. Thus, each camera may detect a wavelength absorbable or emitted, typically emitted, by a respective type or types of molecule (a type of molecule here typically being a chromophore) among said molecules present within the sample, different from the respective types of molecules absorbing or emitting wavelengths of the other cameras. The method also comprises irradiating the sample area with a calibration pattern detectable by each of the cameras. The method also comprises, during the irradiating with the calibration pattern, each of the cameras detecting the calibration pattern from its respective view of the sample area. The method also comprises, based on the detecting of the calibration pattern by each of the cameras, determining rules for transforming respective images acquired by the cameras from the respective views to a primary view. The method also comprises aligning the acquired images of the sample area by using the determined rules.

According to another aspect of the present invention, there is provided an imaging device comprising a controller comprising processing circuitry, and storage storing instructions executable by said processing circuitry whereby said imaging device is operative to perform an embodiment of the method of the present disclosure. The imaging device also comprises an irradiator for irradiating the sample with electromagnetic radiation, and a detector comprising the cameras.

According to another aspect of the present invention, there is provided a computer program product comprising computer-executable components for causing an imaging device, e.g. an embodiment of the imaging device of the present disclosure, to perform an embodiment of the method of the present disclosure when the computer-executable components are run on processing circuitry comprised in the imaging device.

By means of the calibration pattern, which pattern may be known beforehand to the detector so that it knows beforehand what to look for, rules for transforming the images acquired of the sample from the respective sample views of the cameras can be determined and then used for aligning the acquired images of the sample when irradiated with the predetermined pattern. The rules may thus be specific for the sample, accounting for its three-dimensional shape. By the aligning, a point on the surface of the sample, preferably any point, may be identified in each of the acquired images, allowing aligning of the images at that point.

It is to be noted that any feature of any of the aspects may be applied to any other aspect, wherever appropriate. Likewise, any advantage of any of the aspects may apply to any of the other aspects. Other objectives, features and advantages of the enclosed embodiments will be apparent from the following detailed disclosure, from the attached dependent claims as well as from the drawings.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. The use of "first", "second" etc. for different features/components of the present disclosure are only intended to distinguish the features/components from other similar features/components and not to impart any order or hierarchy to the features/components.

### BRIEF DESCRIPTION OF THE DRAWINGS

Embodiments will be described, by way of example, with reference to the accompanying drawings, in which:
Fig 1 is a schematic side view of an imaging device, in accordance with some embodiments of the present invention.
Fig 2 is a schematic bottom view of a detector of an imaging device, in accordance with some embodiments of the present invention.
Fig 3 is a schematic block diagram of a controller of an imaging device, in accordance with some embodiments of the present invention.
Fig 4 is an example image of a predetermined pattern irradiated onto a sample and acquired by a camera in a detector, in accordance with some embodiments of the present invention.
Fig 5 is an example image of a calibration pattern irradiated onto a sample and acquired by a camera in a detector, in accordance with some embodiments of the present invention.
Fig 6 is a schematic flow chart of a method, in accordance with some embodiments of the present invention.

### DETAILED DESCRIPTION

Embodiments will now be described more fully hereinafter with reference to the accompanying drawings, in which certain embodiments are shown. However, other embodiments in many different forms are possible within the scope of the present disclosure. Rather, the following embodiments are provided by way of example so that this disclosure will be thorough and complete, and will fully convey the scope of the disclosure to those skilled in the art. Like numbers refer to like elements throughout the description.

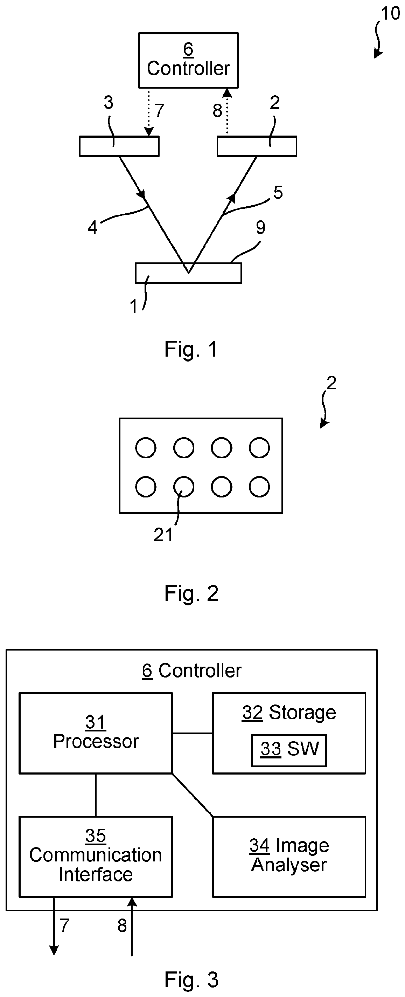

Figure 1 illustrates an imaging device 10 comprising an irradiator 3 and a detector 2. The irradiator may typically be a light source, e.g. comprising a plurality of Light-Emitting Diodes (LED), for instance emitting light 4 in the visible spectrum (VIS) with wavelengths from 380 nm to 750 nm and/or in the near-infrared spectrum (NIR) with wavelengths from 750 nm to 1400 nm. However, other wavelengths of electromagnetic radiation 4 may additionally or alternatively be emitted by the irradiator 3. For instance, at least five or at least eight LEDs may be comprised in the irradiator 3 for emitting the light 4, each with a respective different range of emitted wavelengths. The irradiator 3 preferably emits electromagnetic radiation, preferably VIS and NIR light, covering all the respective wavelength ranges of the cameras 21 in the detector 2 (see figure 2). A projector may be comprised in the irradiator, for projecting the predetermined and calibration patterns onto the sample. To study the inside of the sample 1, and not only the surface 9 thereof, it may be desirable to only detect, by the detector 2, radiation 5 which has been reflected by diffuse reflection, some distance below the surface 9 of the sample, and not detect any radiation reflected by specular reflection at the surface 9. In some embodiments, the irradiator 3 is configured to irradiate the sample 1 with polarized light, e.g. linearly polarized. Since specular reflected radiation keeps its polarization, it may be removed, by a polarization filter or the like, before reaching the sensors, e.g. CCD sensors, of the cameras 21 of the detector 2.

The sample 1 may be a tissue sample, e.g. a human or animal tissue sample, preferably a human tissue sample. In some embodiments, the tissue sample 1 is a sample of live tissue, e.g. part of an arm or leg of a human subject, preferably of a forearm of a human subject.

The imaging device 10 also comprises a controller 6 for controlling the apparatus 10 and for analysing the images acquired by the cameras 21 of the detector 2. Thus, the controller 6 may control the irradiating with predetermined and calibration patterns by the irradiator 3 by sending control signals 7 to the irradiator 3, and the controller may receive image signals 8, comprising the digital images acquired the detector during the irradiating. The controller 6 is further discussed with reference to figure 3.

Figure 2 illustrates a detector 2, e.g. as discussed with reference to figure 1. The detector comprises a plurality of cameras 21, e.g. an array of cameras 21. Since the cameras 21 are arranged beside each other, each has a slightly different view of the sample 1, why the aligning of the present invention is desirable in order to determine where in respective images of the different cameras a point of the sample 1, e.g. a point on the surface 9, is found. Any number of cameras 21 may be comprised in the detector 2, e.g. within the range of 4-10 cameras 21, such as eight cameras as in the figure.

Each camera 21 is configured to detect radiation within a predefined wavelength range specific for that camera, typically distinct or not overlapping with respective wavelength ranges of the other cameras in the detector 2. The radiation detected by each camera is, or at least includes, electromagnetic radiation from diffuse reflection in the sample 1 as a result of the irradiating by the irradiator 3. The specific wavelength ranges may include at least one VIS wavelength range and/or at least one NIR wavelength range, preferably at least one VIS wavelength range and at least one NIR wavelength range. In some embodiments, the specific wavelength ranges include a green VIS wavelength range, a red VIS wavelength range, and at least one NIR wavelength range. To achieve these respective specific wavelength ranges of the cameras 21, each camera may be provided with a bandpass filter corresponding to its specific wavelength range. In some embodiments, the respective bandpass filters of the cameras 21 include bandpass filter(s) having a nominal centre-wavelength value of: 532 nm, 560 nm, 580 nm, 589 nm, 650 nm, 725 nm, 850 nm and/or 975 nm.

As mentioned above, the irradiating by the irradiator 3 may be by polarized light, whereby radiation reflected by specular reflection in the surface 9 of the sample 1 may be removed by a polarization filter, e.g. a polarization filter per camera 21. Thus, in some embodiments, each camera 21 is provided with a respective polarization filter.

In an example, all LEDs of the irradiator 3 radiate simultaneously when irradiating the sample 1 with the predetermined and calibration patterns. With the help of mirrors that reflect or transmit depending on wavelength, a common optical beam path may be formed. The beam may be polarized, typically by linear polarization, and shines on a DMD (digital micromirror device - a projector) which is programmed to reflect the predetermined or calibration pattern. A projector lens focuses the pattern on the sample 1. Each camera 21 has a bandpass filter to acquire images in a specific wavelength range, as well as a polarizing filter to remove specular reflection.

In some embodiments, at least one infrared (IR) camera may be included in the detector 2, e.g. as one of the plurality of camera 21 simultaneously acquiring an image of the sample area. IR may be defined as wavelengths of from 1,4 micrometres (µm) to 15 µm. However, it may not be possible to project the calibration pattern using IR. In that case, the IR camera 21 may include both an IR sensor and a visible light (VIS), or possibly NIR, sensor to form a camera arrangement comprising both an IR sensor and a VIS or NIR sensor. Alternatively, the camera arrangement comprises both an IR camera and a VIS or NIR camera arranged close together, e.g. integrated with each other, such that the IR camera and the VIS/NIR camera has substantially, or at least close to, the same view of the sample 1. In the camera arrangement, the IR sensor may be used for obtaining an IR image of the sample, e.g. during the irradiating with the predetermined pattern, while the VIS/NIR sensor may be used for detecting the calibration pattern for obtaining the rules for aligning the IR image with the respective images acquired by the (other) cameras 21 (e.g. VIS and/or NIR cameras). For instance, the smallest distance between respective lens edges of the lenses for the IR sensor and the VIS/NIR sensor may be at most 1 cm. Thus, in some embodiments of the present invention, at least one of the cameras 21 comprises an IR sensor for acquiring an image in an infrared wavelength range and provides a camera arrangement which also comprises a VIS (or NIR) sensor for detecting the calibration pattern. The IR image may be a simultaneously acquired image 40, acquired during the irradiating of the sample 1 with the predetermined pattern. However, additionally or alternatively, IR image(s) of the sample may be acquired by means of the IR sensor before and/or after the irradiating of the sample with the predetermined pattern. To acquire an IR image before or after, especially before, the irradiating with the predetermined pattern may be suitable to ensure that the IR image captures the naturally radiated heat from the sample and is not affected by the irradiating with the predetermined pattern. In some embodiments, an objective of acquiring an IR image is to look at the naturally radiated heat from the sample 1. If the sample is not irradiated with IR, the acquired IR image may not be impacted by the lower-wavelength (VIS and/or NIR) irradiated predetermined patterns.

Figure 3 illustrates an embodiment of the controller 6. The controller 6 comprises processing circuitry 31 e.g. a central processing unit (CPU). The processing circuitry 31 may comprise one or a plurality of processing units in the form of microprocessor(s). However, other suitable devices with computing capabilities could be comprised in the processing circuitry 31, e.g. an application specific integrated circuit (ASIC), a field programmable gate array (FPGA) or a complex programmable logic device (CPLD). The processing circuitry 31 is configured to run one or several computer program(s) or software (SW) 33 stored in a storage 32 of one or several storage unit(s) e.g. a memory. The storage unit is regarded as a computer readable means 32, forming a computer program product together with the SW 33 stored thereon as computer-executable components and may e.g. be in the form of a Random Access Memory (RAM), a Flash memory or other solid state memory, or a hard disk, or be a combination thereof. The processing circuitry 31 may also be configured to store data in the storage 32, as needed. The controller 6 may also comprise an image analyser 34, e.g. for determining the rules for transforming the images from different cameras to common primary view, and to apply those rules to align the acquired images with each other. The controller 6 may also comprise a communication interface 35, e.g. for communicating with other parts of the imaging device 10. For example, the controller may send control signals 7 via the communication interface 35 and/or receive image signals 8 via the communication interface 35.

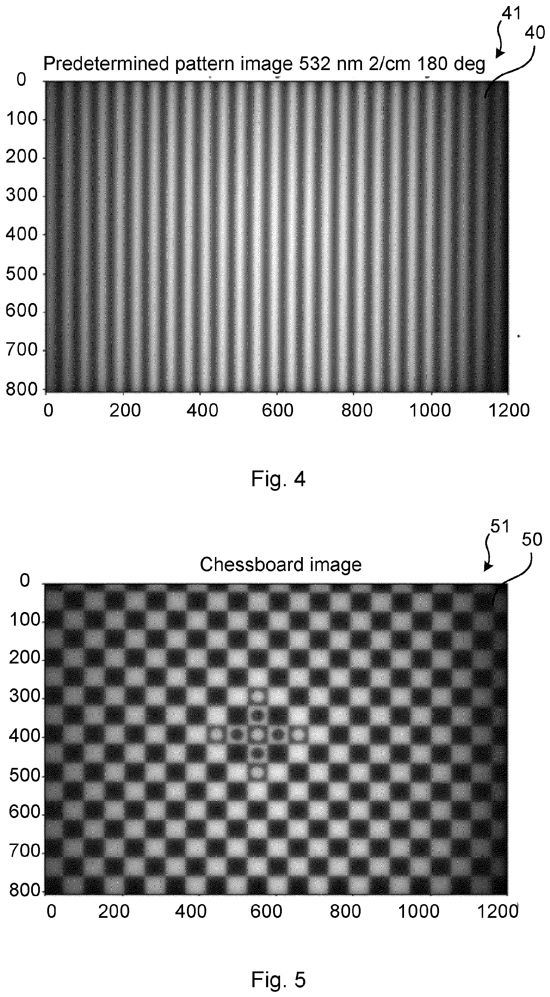

Figure 4 shows an example of an acquired image 40 of a predetermined pattern 41 projected onto the sample 1. The unit on the axes are pixel indices (same for figure 5), whereby in this example row 400 and column 600 is approximately the middle of the image. In this example, a sinusoidal pattern is used. To illuminate the whole sample area, a plurality of predetermined patterns 41 may be needed, e.g. with different phase offsets of the sinusoidal pattern, such as 60°, 180° and 300° offsets, where the pattern 41 having 180° offset is shown in the image 40 of the figure. In addition to its offset, the sinusoidal pattern 41 may also be defined by its spatial frequency, where the pattern 41 of the image 40 in the figure has a frequency corresponding to two wavelengths (or spatial periods) per centimetre (cm) at a standard distance of the sample from the irradiator/projector 3, when irradiating a planar surface 9 of the sample 1 at a right angle. In the example of figure 4, the image 40 is acquired by a camera 21 having a bandpass filter with a nominal centre-wavelength value of 532 nanometres (nm).

Figure 5 shows an example of an acquired image 50 of a calibration pattern 50, here in the form of a chessboard pattern. The calibration pattern is projected onto the sample with radiation including wavelengths such that the pattern is detectable by all of the cameras 21 in the detector 2. It is preferable to use a calibration pattern 51 without features such as dots, speckles or (thin) lines which may be confused with physiological features like hair, freckles or skin folds. For instance, a dot could be confused with a naturally occurring skin pigment spot, and a line of e.g. a grid pattern could be confused with a body hair. It may be convenient to use a calibration pattern including filled geometrical symbols/figures, e.g. squares, rectangles, triangles, circles, plus signs, rings and/or crosses, or the like, e.g. with a filled thickness of at least 1, 5 or 10 mm to prevent confusion with body hair. A currently preferred calibration pattern is a chessboard pattern of alternating filled and non-filled squares. Another currently preferred pattern is a predefined 2-dimensional matrix of mixed symbols such as including squares, triangles and plus signs, preferably filled. A calibration pattern with more than one different filled symbol may be advantageous if the surface 9 of the sample 1 is not flat, e.g. if the sample is in the face of the subject, in which case it may be difficult e.g. in a chessboard pattern to determine which of the squares is which (e.g. if not all squares are readily visible to the cameras 21).

The calibration pattern 51 is configured such that it is possible to align images of the pattern from each of the different cameras, from their respective views of the sample area. For instance, the calibration pattern may include a feature which is readily detectable and may serve as an origin for determining coordinates within the respective images 50. In the example of figure 5, a central cross with circles serves as such a feature within the chessboard pattern 51. Starting from the origin, coordinates may be assigned to other features in the calibration pattern 51, e.g. corners in the chessboard pattern of figure 5. Optionally, a distinct ID may be assigned to each feature/corner. Each respective image 50 of the calibration pattern 51 detected by the cameras 21 is analysed to identify the features therein, e.g. with coordinates or IDs. Then, rules, specific for the sample 1, can be determined for transforming respective images 50 or 40 acquired by each camera 21 from its view of the sample to a common primary view. Then, the determined rules can be used for aligning the images 40 of the predetermined pattern 41, e.g. comprising Piecewise Affine Transform.

For example, coordinates in the calibration pattern 51, in the form of a chessboard pattern as in figure 5, correspond to the corner locations in the chessboard pattern, so there is a certain spacing between coordinates, which may e.g. be >10 pixels in the images 50. Therefore, there are typically not corresponding coordinates for each pixel in each camera view, why there may be a need to approximate a transform between coordinates. The piecewise affine transform may perform adequately for this. In theory, however, coordinates for every pixel in the image 50 may be determined, allowing each pixel in the acquired images 40 to be aligned with each other.

In an example image alignment method, a calibration pattern 51 is projected onto the sample area. The calibration pattern could be a chessboard pattern or a pattern with mixed symbols as discussed above, such as squares, triangles and plus signs, preferably filled. For the camera view of each of the cameras 21 detecting S4 the calibration pattern, a matched filter that enhances the location of calibration pattern features may be applied. Features are for example the chessboard squares' corners or the symbols mentioned previously. The resulting filtered image will have high values ("peaks") at the feature locations. Coordinates of the features may be determined by, for example, thresholding the filtered image and calculating the centre of gravity within a window that moves across the full filtered image. Between camera views of the different cameras 21, e.g. between a primary camera view and each respective view of the other cameras, corresponding feature locations may be determined by for example iterative closest point registration. Based on the locations of corresponding features between camera views, a piecewise affine transform can be determined, such that the feature locations align after the transformation.

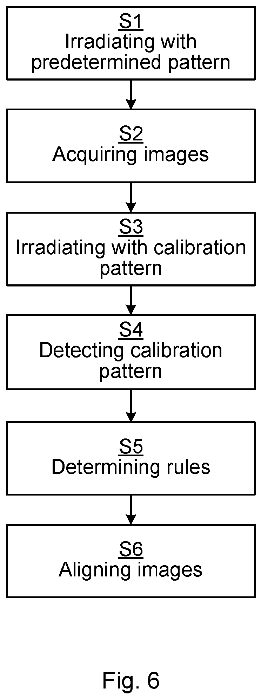

Figure 6 illustrates some embodiments of the method of the present disclosure. The method is for aligning simultaneously acquired images 40 of the sample 1 while it is irradiated with a predetermined pattern 41 projected onto the sample. An area of the sample 1 is irradiated S1 with electromagnetic radiation 4. In some embodiments, the radiation 4 includes wavelengths absorbable by molecules present within the sample, below a surface 9 of the sample. The sample may be a tissue sample, in which case the molecules may be chromophores present in tissue, such as water, melanin, oxygenated haemoglobin and/or unoxygenated haemoglobin. Typically, the predetermined pattern 41 is static during the acquiring of images thereof. During the irradiating S1 of the sample 1 with the predetermined pattern 41, an image 40 of the sample area is acquired S2 by each of a plurality of cameras 21 simultaneously in a wavelength range specific to that camera. For instance, each of the cameras 21 may acquire its image 40 over the whole of its specific wavelength range, wherein said wavelength range comprises wavelength(s) absorbed or emitted by at least one of the chromophores in the sample. Thus, the presence and amount present of said chromophores in the sample may be detected and measured in the images 40. Before or after the irradiating S1 with the predetermined pattern, the sample area is irradiated S3 with a calibration pattern 51 detectable by each of the cameras 21. The calibration patten may be projected onto a surface 9 of the sample. The calibration pattern is then detected S4, e.g. by acquiring an image thereof, by each of the cameras 21 from its respective view of the sample area (resulting from its viewing angle of the sample) during the irradiating S3 with the calibration pattern 51. Thus, the same pattern is detected S4 in parallel by the cameras 21. Based on the detecting S4 of the calibration pattern 51 by each of the cameras 21, rules are determined S5 for transforming the respective images 40, acquired S2 by the cameras 21, from the respective views to a primary view. Then, the determined S5 rules are applied to align S6 the acquired S2 images 40 of the sample area.

The present disclosure has mainly been described above with reference to a few embodiments. However, as is readily appreciated by a person skilled in the art, other embodiments than the ones disclosed above are equally possible within the scope of the present disclosure, as defined by the appended claims.

## Claims

1. A computer-implemented method of aligning simultaneously acquired images (40) of a sample (1) irradiated with a predetermined pattern (41), the method comprising:
with said predetermined pattern (41), irradiating (S1) an area of the sample (1) with electromagnetic radiation (4) including wavelengths absorbable by molecules present within the sample, below a surface (9) of the sample;
by each of a plurality of cameras (21) simultaneously, during the irradiating (S1) of the sample (1) with the predetermined pattern (41), acquiring (S2) an image (40) of the sample area in a wavelength range specific to that camera and different from the respective wavelength ranges of the other cameras;
irradiating (S3) the sample area with a calibration pattern (51) detectable by each of the cameras (21);
during the irradiating (S3) with the calibration pattern (51), each of the cameras (21) detecting (S4) the calibration pattern from its respective view of the sample area;
based on the detecting (S4) of the calibration pattern (51) by each of the cameras (21), determining (S5) rules for transforming respective images (40) acquired (S2) by the cameras from the respective views to a primary view; and
aligning (S6) the acquired (S2) images (40) of the sample area by using the determined (S5) rules, wherein the aligning (S6) comprises Piecewise Affine Transform;
wherein at least one of the cameras (21) is an IR camera, said IR camera comprising an IR sensor acquiring (S2) the image of the sample area during the irradiating (S1) of the sample with the predetermined pattern (41) and a VIS or NIR sensor detecting (S4) the calibration pattern during the irradiating (S3) with the calibration pattern (51).

2. The method of claim 1, wherein the sample (1) is a tissue sample, e.g. a human or animal tissue sample, preferably a human tissue sample.

3. The method of claim 2, wherein the tissue sample (1) is a live tissue sample.

4. The method of any preceding claim, wherein the calibration pattern includes filled geometrical symbols e.g. squares, rectangles, triangles, circles, plus signs, rings and/or crosses.

5. The method of claim 4, wherein the calibration pattern is a chessboard pattern or a pattern of mixed different symbols in a 2-dimensional matrix.

6. The method of claim 4 or 5, wherein each of the symbols has a filled thickness of at least 1, 5 or 10 mm.

7. The method of any preceding claim, wherein the plurality of cameras (21) are within the range of 4-10 cameras, preferably eight cameras.

8. The method of any preceding claim, wherein the respective specific wavelength ranges of the cameras (21) include at least one wavelength range in the visual spectrum, VIS, and at least one wavelength range in the near-infrared spectrum, NIR.

9. The method of any preceding claim, wherein each of the cameras (21) is provided with a bandpass filter corresponding to its specific wavelength range.

10. The method of claim 9, wherein the respective bandpass filters of the cameras (21) include bandpass filter(s) having a nominal centre-wavelength value of: 532 nm, 560 nm, 580 nm, 589 nm, 650 nm, 725 nm, 850 nm and/or 975 nm.

11. The method of any preceding claim, wherein the plurality of cameras (21) are comprised in a detector (2) which also comprises at least one camera arrangement comprising both an IR sensor for acquiring an IR image of the sample area and a VIS or NIR, preferably VIS, sensor for detecting the calibration pattern (51).

12. The method of any preceding claim, wherein the irradiating (S1) with the predetermined pattern (41) and the irradiating (S3) with the calibration pattern (51) is by a plurality of LEDs emitting electromagnetic radiation (4) in respective different wavelength ranges.

13. The method of any preceding claim, wherein the irradiating (S1) with the predetermined pattern (41) and the irradiating (S3) with the calibration pattern (51) is by polarized light (4).

14. The method of claim 13, wherein at least one, e.g. some or all, of the cameras (21) is provided with a polarization filter.

15. An imaging device (10) comprising:
a controller (6) comprising processing circuitry (31), and storage (32) storing instructions (33) executable by said processing circuitry whereby said imaging device is operative to perform the method of any preceding claim;
an irradiator (3) for irradiating the sample (1) with electromagnetic radiation (4); and
a detector (2) comprising the cameras (21).

16. A computer program product (32) comprising computer-executable components (33) for causing an imaging device (10) to perform the method of any one of claims 1-14 when the computer-executable components are run on processing circuitry (31) comprised in the imaging device.

## Patentansprüche

1. Computerimplementiertes Verfahren zum Ausrichten von gleichzeitig aufgenommenen Bildern (40) einer Probe (1), die mit einem vorbestimmten Muster (41) bestrahlt wird, wobei das Verfahren Folgendes umfasst:
mit dem vorbestimmten Muster (41), Bestrahlen (S1) eines Bereichs der Probe (1) mit elektromagnetischer Strahlung (4), die Wellenlängen einschließt, die durch Moleküle absorbierbar sind, die innerhalb der Probe vorhanden sind, unterhalb einer Oberfläche (9) der Probe,
durch jede von einer Vielzahl von Kameras (21) gleichzeitig, während des Bestrahlens (S1) der Probe (1) mit dem vorbestimmten Muster (41), Aufnehmen (S2) eines Bildes (40) des Probenbereichs in einem Wellenlängenbereich, der für diese Kamera spezifisch ist und sich von den jeweiligen Wellenlängenbereichen der anderen Kameras unterscheidet,
Bestrahlen (S3) des Probenbereichs mit einem Kalibrierungsmuster (51), das durch jede von den Kameras (21) erfassbar ist,
während des Bestrahlens (S3) mit dem Kalibrierungsmuster (51), durch jede von den Kameras (21), Erfassen (S4) des Kalibrierungsmusters aus ihrer jeweiligen Ansicht des Probenbereichs,
auf Grundlage des Erfassens (S4) des Kalibrierungsmusters (51) durch jede von den Kameras (21), Bestimmen (S5) von Regeln zum Umformen von jeweiligen Bildern (40), die durch die Kameras aus den jeweiligen Ansichten aufgenommen (S2) wurden, zu einer primären Ansicht und
Ausrichten (S6) der aufgenommenen (S2) Bilder (40) des Probenbereichs durch Verwenden der bestimmten (S5) Regeln, wobei das Ausrichten (S6) eine stückweise affine Transformation umfasst,
wobei mindestens eine von den Kameras (21) eine IR-Kamera ist, wobei die IR-Kamera einen IR-Sensor, der das Bild des Probenbereichs während des Bestrahlens (S1) der Probe mit dem vorbestimmten Muster (41) aufnimmt (S2), und einen VIS- oder NIR-Sensor, der das Kalibrierungsmuster während des Bestrahlens (S3) mit dem Kalibrierungsmuster (51) erfasst (S4), umfasst.

2. Verfahren nach Anspruch 1, wobei die Probe (1) eine Gewebeprobe, z. B. eine menschliche oder tierische Gewebeprobe, vorzugsweise eine menschliche Gewebeprobe, ist.

3. Verfahren nach Anspruch 2, wobei die Probe (1) eine lebende Gewebeprobe ist.

4. Verfahren nach einem der vorhergehenden Ansprüche, wobei das Kalibrierungsmuster gefüllte geometrische Symbole, z. B. Quadrate, Rechtecke, Dreiecke, Kreise, Pluszeichen, Ringe und/oder Kreuze, einschließt.

5. Verfahren nach Anspruch 4, wobei das Kalibrierungsmuster ein Schachbrettmuster oder ein Muster von gemischten unterschiedlichen Symbolen in einer 2-dimensionalen Matrix ist.

6. Verfahren nach Anspruch 4 oder 5, wobei jedes von den Symbolen eine gefüllte Dicke von mindestens 1,5 oder 10 mm aufweist.

7. Verfahren nach einem der vorhergehenden Ansprüche, wobei die Vielzahl von Kameras (21) innerhalb des Bereichs von 4 bis 10 Kameras, vorzugsweise acht Kameras, liegt.

8. Verfahren nach einem der vorhergehenden Ansprüche, wobei die jeweiligen spezifischen Wellenlängenbereiche der Kameras (21) mindestens einen Wellenlängenbereich in dem visuellen Spektrum, VIS, und mindestens einen Wellenlängenbereich in dem Nahe-Infrarot-Spektrum, NIR, einschließen.

9. Verfahren nach einem der vorhergehenden Ansprüche, wobei jede von den Kameras (21) mit einem Bandpassfilter versehen ist, der ihrem spezifischen Wellenlängenbereich entspricht.

10. Verfahren nach Anspruch 9, wobei die jeweiligen Bandpassfilter der Kameras (21) (einen) Bandpassfilter einschließen, der/die einen nominellen Mittenwellenlängenwert von: 532 nm, 560 nm, 580 nm, 589 nm, 650 nm, 725 nm, 850 nm und/oder 975 nm aufweist/aufweisen.

11. Verfahren nach einem der vorhergehenden Ansprüche, wobei die Vielzahl von Kameras (21) in einem Detektor (2) enthalten sind, der ebenfalls mindestens eine Kameraanordnung umfasst, die sowohl einen IR-Sensor zum Aufnehmen eines IR-Bildes des Probenbereichs als auch einen VIS- oder NIR-, vorzugweise VIS-, Sensor zum Erfassen des Kalibrierungsmusters (51) umfasst.

12. Verfahren nach einem der vorhergehenden Ansprüche, wobei das Bestrahlen (S1) mit dem vorbestimmten Muster (41) und das Bestrahlen (S3) mit dem Kalibrierungsmuster (51) durch eine Vielzahl von LED, die elektromagnetische Strahlung (4) in jeweiligen unterschiedlichen Wellenlängenbereichen emittieren, erfolgen.

13. Verfahren nach einem der vorhergehenden Ansprüche, wobei das Bestrahlen (S1) mit dem vorbestimmten Muster (41) und das Bestrahlen (S3) mit dem Kalibrierungsmuster (51) durch polarisiertes Licht (4) erfolgen.

14. Verfahren nach Anspruch 13, wobei mindestens eine, z. B. einige oder alle, von den Kameras (21) mit einem Polarisationsfilter versehen ist/sind.

15. Bildgebungseinrichtung (10), die Folgendes umfasst:
eine Steuereinrichtung (6), umfassend Verarbeitungsschalttechnik (31) und Speicher (32), der Anweisungen (33) speichert, die durch die Verarbeitungsschalttechnik ausführbar sind, wodurch die Bildgebungseinrichtung funktionsfähig ist, um das Verfahren nach einem der vorhergehenden Ansprüche durchzuführen,
eine Bestrahlungsvorrichtung (3) zum Bestrahlen der Probe (1) mit elektromagnetischer Strahlung (4) und
einen Detektor (2), der die Kameras (21) umfasst.

16. Computerprogrammprodukt (32), das computerausführbare Komponenten (33) umfasst, zum Veranlassen, dass eine Bildgebungseinrichtung (10) das Verfahren nach einem der Ansprüche 1 bis 14 durchführt, wenn die computerausführbaren Komponenten auf Verarbeitungsschalttechnik (31), die in der Bildgebungseinrichtung enthalten ist, laufen gelassen werden.

## Revendications

1. Procédé mis en œuvre par ordinateur pour aligner des images (40) acquises simultanément d'un échantillon (1) irradié avec un modèle prédéfini (41), procédé comprenant :
avec ledit modèle prédéfini (41), irradiation (S1) d'une zone de l'échantillon (1) avec un rayonnement électromagnétique (4) comportant des longueurs d'ondes pouvant être absorbées par des molécules présentes à l'intérieur de l'échantillon, sous une surface (9) de l'échantillon ;
par chacune d'une pluralité de caméras (21) simultanément, pendant l'irradiation (S1) de l'échantillon (1) avec le modèle prédéfini (41), acquisition (S2) d'une image (40) de la zone d'échantillon dans une plage de longueurs d'ondes spécifique à cette caméra et différente des plages de longueurs d'ondes respectives des autres caméras ;
irradiation (S3) de la zone d'échantillon avec un modèle d'étalonnage (51) pouvant être détecté par chacune des caméras (21) ;
pendant l'irradiation (S3) avec le modèle d'étalonnage (51), détection (S4) par chacune des caméras (21) du modèle d'étalonnage depuis leur vue respective sur la zone d'échantillon ;
sur la base de la détection (S4) du modèle d'étalonnage (51) par chacune des caméras (21), détermination (S5) de règles pour transformer des images (40) respectives acquises (S2) par les caméras depuis leurs vues respectives en une vue primaire ; et
alignement (S6) des images (40) acquises (S2) de la zone d'échantillon à l'aide des règles déterminées (S5), dans lequel l'alignement (S6) comprend une transformation affine par morceaux ;
dans lequel au moins une des caméras (21) est une caméra infrarouge (IR), ladite caméra IR comprenant un capteur IR acquérant (S2) l'image de la zone d'échantillon pendant l'irradiation (S1) de l'échantillon avec le modèle prédéfini (41) et un capteur de spectrométrie visible (VIS) ou proche infrarouge (NIR) détectant (S4) le modèle d'étalonnage pendant l'irradiation (S3) avec le modèle d'étalonnage (51).

2. Procédé selon la revendication 1, dans lequel l'échantillon (1) est un échantillon de tissu, p. ex. un échantillon de tissu humain ou animal, de préférence un échantillon de tissu humain.

3. Procédé selon la revendication 2, dans lequel l'échantillon de tissu (1) est un échantillon de tissu vivant.

4. Procédé selon l'une quelconque des revendications précédentes, dans lequel le modèle d'étalonnage comporte des symboles géométriques pleins, p. ex. des carrés, des rectangles, des triangles, des cercles, des signes plus, des anneaux et/ou des croix.

5. Procédé selon la revendication 4, dans lequel le modèle d'étalonnage est un modèle de damier ou un modèle de différents symboles mélangés dans une matrice bidimensionnelle.

6. Procédé selon la revendication 4 ou 5, dans lequel chacun des symboles a une épaisseur pleine d'au moins 1,5 ou 10 mm.

7. Procédé selon l'une quelconque des revendications précédentes, dans lequel la pluralité de caméras (21) se situe dans une plage de 4-10 caméras, de préférence huit caméras.

8. Procédé selon l'une quelconque des revendications précédentes, dans lequel les plages spécifiques de longueurs d'ondes respectives des caméras (21) comportent au moins une plage de longueurs d'ondes dans le spectre visible, VIS, et au moins une plage de longueurs d'ondes dans le spectre proche infrarouge, NIR.

9. Procédé selon l'une quelconque des revendications précédentes, dans lequel chacune des caméras (21) est munie d'un filtre passe-bande correspondant à sa plage spécifique de longueurs d'ondes.

10. Procédé selon la revendication 9, dans lequel les filtres passe-bande respectifs des caméras (21) comportent un (des) filtre(s) passe-bande ayant une valeur nominale de longueur d'onde centrale de : 532 nm, 560 nm, 580 nm, 589 nm, 650 nm, 725 nm, 850 nm et/ou 975 nm.

11. Procédé selon l'une quelconque des revendications précédentes, dans lequel la pluralité de caméras (21) est comprise dans un détecteur (2) qui comprend également au moins un dispositif de caméra comprenant un capteur IR pour acquérir une image IR de la zone d'échantillon et un capteur VIS ou NIR, de préférence VIS, pour détecter le modèle d'étalonnage (51).

12. Procédé selon l'une quelconque des revendications précédentes, dans lequel l'irradiation (S1) avec le modèle prédéfini (41) et l'irradiation (S3) avec le modèle d'étalonnage (51) s'effectue par une pluralité de LED émettant un rayonnement électromagnétique (4) dans différentes plages de longueurs d'ondes respectives.

13. Procédé selon l'une quelconque des revendications précédentes, dans lequel l'irradiation (S1) avec le modèle prédéfini (41) et l'irradiation (S3) avec le modèle d'étalonnage (51) s'effectue par lumière polarisée (4).

14. Procédé selon la revendication 13, dans lequel au moins une, p. ex. quelques-unes ou toutes, des caméras (21) est munie d'un filtre de polarisation.

15. Dispositif d'imagerie (10) comprenant :
un contrôleur (6) comprenant des circuits de traitement (31) et un stockage (32) stockant des instructions (33) exécutables par lesdits circuits de traitement, selon lequel ledit dispositif d'imagerie sert à exécuter le procédé selon l'une quelconque des revendications précédentes ;
un irradiateur (3) pour irradier l'échantillon (1) avec un rayonnement électromagnétique (4) ; et
un détecteur (2) comprenant les caméras (21).

16. Produit programme informatique (32) comprenant des composants exécutables par ordinateur (33) pour amener un dispositif d'imagerie (10) à exécuter le procédé selon l'une quelconque des revendications 1-14 lorsque les composants exécutables par ordinateur sont exécutés sur des circuits de traitement (31) compris dans le dispositif d'imagerie.
